# EUROPEAN PATENT APPLICATION

(11) **EP 0 862 901 A1**
(43) Date of publication of application: **09.09.1998**
(21) Application number: 98300248.6
(22) Date of filing: 14.01.1998
(51) Int. Cl.: A61F 7/08, A61G 11/00

(54) **Thermoelectric infant mattress**

(30) Priority: 05.03.1997 US 811975
(71) Applicant: OHMEDA INC., Liberty Corner, New Jersey 07938-0804 (US)
(72) Inventor: Sweeney, Stephen J., Sykesville, Maryland 21784 (US); Spencer, Timothy A., New Windsor, Maryland 21776 (US)
(74) Representative: Hedley, Nicholas James Matthew

(57) **Abstract**

A mattress assembly (26) for use in the heating and/or cooling of a patient (18), particularly an infant, wherein one or more thermoelectric devices (28) are used to heat the mattress (26) filled with a heat conducting material. The patient (18) can thus be readily heated or cooled as desired by the user with only minimal disruption to the patient care apparatus (10) being used. As a further embodiment, a plurality of thermoelectric devices (28) are utilized and which allow simultaneous compartmentalized heating and/or cooling of the patient (18) so that the user can set differing temperatures for differing parts of the body of the patient (18).

## Description

This invention relates to a mattress for the heating and/or cooling of a patient, and, more particularly, to the heating and/or cooling of an infant contained within an infant care apparatus.

In general, certain patients, particularly infants, require considerable temperature control and typically, many newborns are provided with heat through overhead heaters, an example of which is shown and described in U.S. Patent 5,474,517. Alternatively, the infant may be heated by means of a specially controlled environment provided by an infant incubator such as is shown and described in U.S. Patent 4,936,834.

One difficulty of such systems , however, is that there are occasions when it is desirable to cool the infant, particularly a part thereof. Obviously, in the case of cooling, there is no viable overhead cooling systems and therefore the cooling must take place within the closed or semiclosed environment within which the infant is placed.

A further problem arises in the care of infants in that it is sometimes advantageous to provide both cooling and heating of the infant simultaneously, such as where the infant's head is cooled while the remaining surface of the infant is heated.

One means of providing heat and cooling to a patient is by means of a heated or cooled mattress as shown and described in U.S. 5,448,788. In that patent, a heated and cooled mattress underlies the patient and circulates a liquid, such as water, continuously through the mattress. The temperature of the water is externally controlled so as to affect the temperature of the mattress and, of course the patient in that heating or cooling is passed on to the patient.

The use of a circulating fluid, such as water is, however, cumbersome to the apparatus and particularly in the case of an infant incubator, causes considerable additional design and expense to the overall apparatus. Infant incubators do not normally provide for heated or cooled circulating water and therefore, to add that type of temperature regulation to an infant incubator would require extensive redesign at considerable cost.

Other means of providing heating and/or cooling to an infant is known where the circulating air within the infant compartment is cooled or heated. An example is in U.S. Patent 4,458,674 where a non-electrical means in inserted into the incubator to either heat or cool the air that circulates through that compartment. Other circulating means include the use of ice to provide cooling to the air circulating in the infant compartment as in U.S. 3,076,451.

The use of the foregoing methods, however, have drawbacks, for example, the addition of ice or other cooling means to the circulating air systems in an incubator is, again, cumbersome, and, of course, only has limited operating times after which the ice must be replaced, causing additional maintenance needs. In addition, such systems are not able to make rapid changes in the heating or cooling and which is advantageous in the case of an infant incubator, to be able to exercise relatively rapid changes in the temperatures such as when a incubator door is opened and the use of a circulating fluid makes such rapid changes quite difficult.

Lastly, the aforementioned systems are not capable of being usable in those situations where it is advantageous to cool one portion of the infant's body while simultaneously heating another portion of the body.

According to one aspect of the present invention, a mattress assembly adapted to underlie a patient and to provide thermal regulation to the patient comprises; a mattress containing a heat transfer medium, a thermoelectric in heat transfer relationship with said mattress and said heat transfer medium and control means activating said thermoelectric device to cause said thermoelectric device to affect the temperature of said mattress

A heated and/or cooled mattress is provided that is particularly adapted for patients, such as infants contained within an infant incubator, and which can be controlled with relative ease and little additional control devices and yet which can fairly rapidly respond to the particular condition desired by the user.

The mattress of the present invention is heated and/or cooled by means of thermoelectric devices and which provide the needed heat or cooling to the infant rapidly without causing unneeded increased cost and complexity to the overall infant apparatus that may contain the infant. A typical use of the present mattress would be within the infant apparatus, such as a infant incubator.

According to a further aspect of the present invention, a method of heating and cooling an infant comprises the steps of:
providing a mattress underlying and supporting the infant containing a heat conducting material;
providing a thermoelectric device in heat transfer relationship with the mattress and the heat conducting material;
providing an electrical circuit for causing the thermoelectric device to provide temperature control to the mattress; and
controlling the electric circuit to cause the thermoelectric device to cause the mattress to reach a desired temperature.

With the use of a mattress that is directly heated by the thermoelectric device, it is possible to readily cool the infant within the incubator without the need for circulating fluids for cooling the stream of air that is introduced into the infant compartment. Thus, the thermoelectric cooled mattress may be readily added to present infant incubators by the addition of a relatively simple electrical circuit and extensive, complicated modifications to the infant incubator are avoided.

As a further enhancement with the use of a thermoelectric device cooled/heated mattress, the thermoelectric device is readily capable of compartmentalized cooling and heating, that is, the thermoelectric device can simultaneously heat and cool different areas of the infant so that certain therapy may be provided as desired by the user.

Embodiments of the invention will now be described, by way of example, reference being made to the Figures of the accompanying diagrammatic drawings in which:-
Figure 1 is a end schematic view of a thermoelectric heated/cooled mattress constructed in accordance with the present invention contained within an infant incubator;
Figure 2 is a side view of the invention of FIG. 1;
Figure 3 is a schematic view of a mattress heated and or cooled by a thermoelectric device showing the control scheme; and
Figure 4 is a schematic view of a compartmentalized thermoelectric heated/cooled mattress incorporating the present invention.

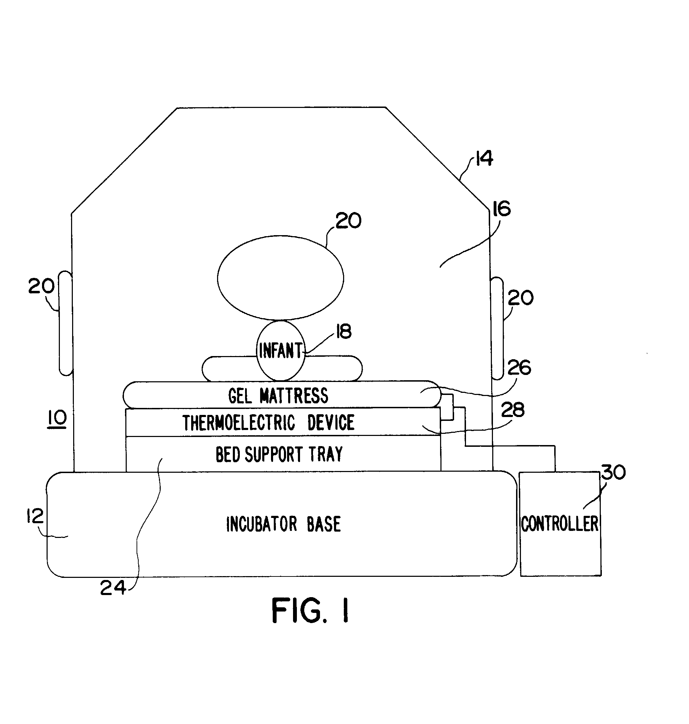

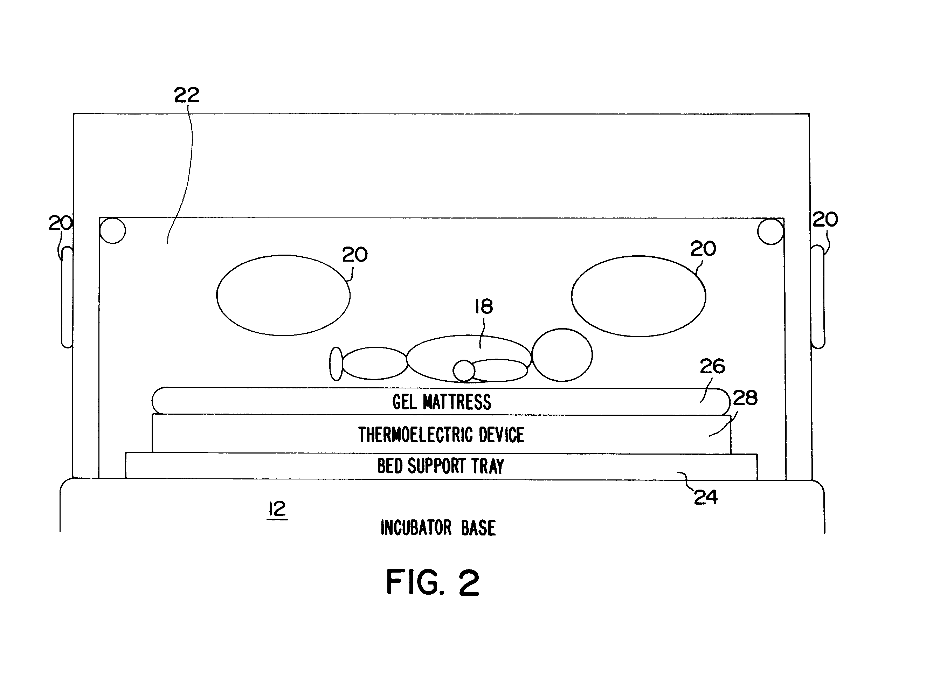

Turning now to Figure 1 and Figure 2 there is shown a schematic end view and a side view, respectively, of an incubator 10 having a mattress assembly constructed in accordance with the present invention. The incubator 10 may be of conventional construction and one example of an incubator suitable for use with the present invention is shown and described in U.S. Patent 4,936,824 and assigned to the present assignee.

As can be seen, the incubator 10 comprises a base 12 and which generally contains the mechanical apparatus used to circulate and heat the warm air that is used to heat the internal infant compartment 14 of the incubator 10. A hood 16 generally sits upon the base and encloses the infant compartment 14 where the infant 18 is positioned. The hood 16 is of a transparent material to provide visual monitoring of the infant 18 and also may have various handholes 20 to provide access to the infant 18 and a front door 22 that is used to place the infant 18 into the infant compartment 14 and to remove it therefrom as well as to provide full access to the infant 18 to carry out various procedures in caring for the infant 18.

A bed support tray 24 is provided in the infant compartment 14 resting, generally on the base 12 to support the infant 18. A mattress 26 underlies the infant 18 to provide comfort and support to the infant 18. As will be seen, the mattress 26 contains a heat conductive material that readily conducts heat to and from the infant 18. One suitable heat conductive material that is usable with the present invention is a silicon or polyurethane gel.

A thermoelectric device 28 is located in heat conductive relationship with the mattress 26 and, as shown, the thermoelectric device 28 is positioned beneath the mattress 26 with respect to the infant 18 so that the thermoelectric device 28 can readily provide heat or remove heat from the mattress 26 directly. The thermoelectric device 28 could be placed in other positions, however, in the preferred embodiment, the device is sized to be about the same area as the mattress and underlies that mattress 26.

The use of a thermoelectric device 28 in the present invention is believed key to the overall purpose of the invention. Such devices are readily available commercially, one such supplier being the Materials Electronic Products Corporation of Trenton, NJ and the devices are basically static devices that are heat pumps containing a plurality of thermoelectric cooling couples made from two elements, primarily Bismuth Telluride, heavily doped to create either an excess (N type) or deficiency (P type) of electrons. Heat absorbed at the cold junction is pumped to the hot junction at a rate proportional to the carrier current passing through the circuit and the number of couples.

Thus, the conventional thermoelectric device is responsive to the current to produce a cold junction and a hot junction. In the present invention, therefore, by intermingling both the hot and cold junctions, it is possible to provide both cooling and heating to the mattress 26 that is compartmentalized to cool and/or heat segments of the mattress as desired by the operator. Thus, in the event the infant 18 is overheated, the thermoelectric device 28 can rapidly cool the infant to the desired temperature by simply employing the cold junctions to provide an active cooling rather than merely wait for the infant to lower its temperature through a cooler ambient temperature. Therefore, better overall temperature control can be exercised by the device over the infant mattress as opposed to devices that do not include the cooling function.

Finally, in the Figure 1, there is included a controller 30 that controls the heating and/or cooling of the thermoelectric device 28 and, obviously, also the infant 18. The controller 30 is, again, a conventional controller and is connected by suitable wires to a temperature sensor, (not shown in Figure 1) in the mattress 26 and to the thermoelectric device 28. Accordingly, the controller 30 can sense the temperature of the mattress 26 and, depending upon a desired set point, can send a signal to control the thermoelectric device 28 to attain and maintain that desired temperature. The desired input temperature may be inputted to the controller 30 by a keyboard, dial, or the like.

As can be seen in Figure 1, the addition of the controller 30 requires a relatively minimal change to the overall incubator 10 and which thereby allows certain cooling and heating of the infant 18. By the use of a thermoelectric device, the changes to the incubator are simply accomplished as opposed to various systems that provide mattresses that are cooled or heated by flowing streams of air or a liquid. In such air or liquid circulating systems, the overall incubator 10 would require the source of, for example, a liquid, pumping equipment, heating and cooling equipment and considerable other modifications to provide such a system in the incubator.

Accordingly, it is the use of a thermoelectric device 28 that enables both the heating and cooling of the infant without totally revamping the infant care apparatus.

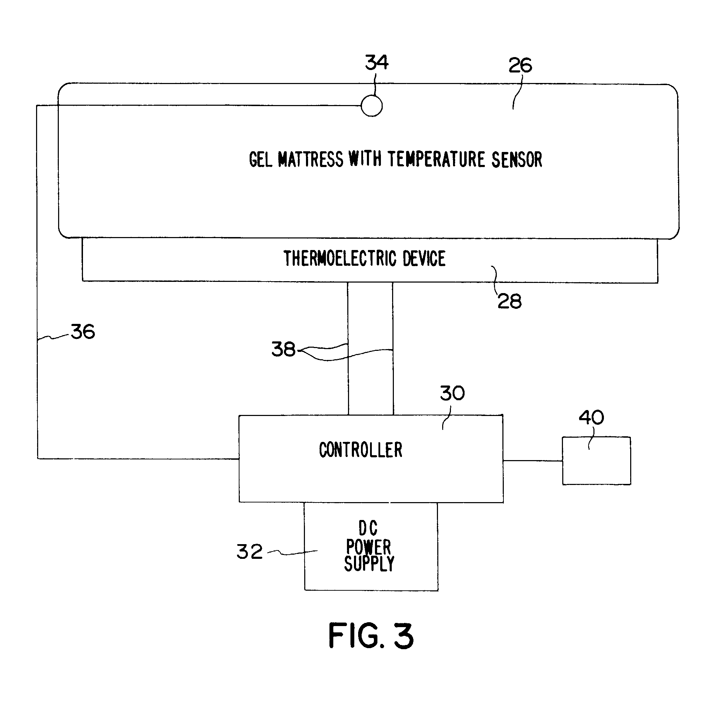

Turning now to Figure 3, there is shown a schematic view of the control system that can be used in carrying out the present invention. As can be seen, the controller 30 is powered by a DC power supply 32 of conventional design and a temperature sensor 34 is located in the mattress 26 to continually sense the temperature of that mattress 26. The temperature sensor 34 is, of course electrically communicating with the controller 30 by means of a cable 36 so that the temperature of the mattress 26 is continually fed to the controller 30. As also can be seen, various cables 38 connect the controller 30 to the thermoelectric device 28 to provide the current to effect either the cooling function or the heating function of the thermoelectric device 28.

Finally, an input device 40 is provided so as to allow the user to set a desired temperature for the thermoelectric device 28. The input device 40 may, of course, be a part of the controller 30, or may be a separate device such as a keyboard, dial, digital input or the like. Basically, the user accesses the input device 40 to set the desired temperature for the mattress 26. The controller 30 thus activates the thermoelectric device 28 to either provide heating or cooling to the mattress to achieve that desired set temperature and the temperature sensor 34 continually senses that temperature and feeds the data back to the controller so that the controller can recognize when the desired set temperature has been achieved.

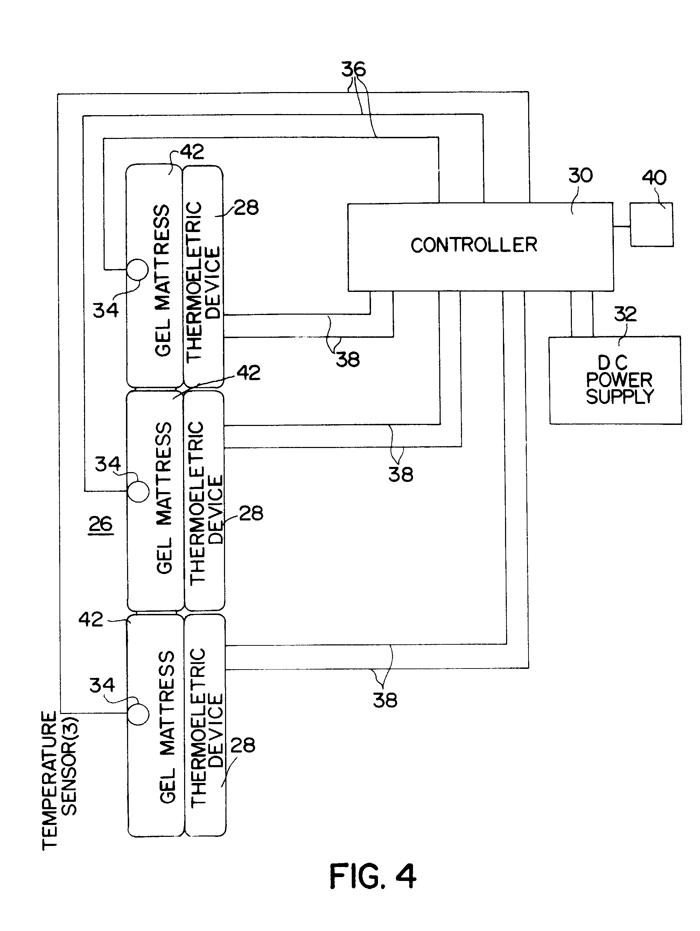

Turning now to FIG. 4, there is shown a schematic view of a plurality of compartmentalized mattress segments 42 that combine to form a mattress as shown in Fig.'s 1-3. In FIG. 4, the normal mattress 26 that underlies an infant, is compartmentalized into mattress segments 42 so that individual mattress segments 42 can affect differing temperatures to the infant. As shown, each mattress segment is in heat conducting relationship with a separate thermoelectric device 28 so that each mattress segment 42 can be set to a different temperature from the other mattress segments 42. As previously explained, it is sometimes beneficial in the care of infants that the head of the infant be maintained cool while providing heat to the remaining body portions. With the FIG. 4 embodiment, each mattress segment 42 has a separate temperature sensor 34 such that each is heated or cooled individually by a thermoelectric device 28.

Thus, various parts of the infant or patient can be regulated to differing temperatures, either by heating or cooling, through separate input devices 40 for more complete thermoregulation of the patient.

## Claims

1. A mattress assembly adapted to underlie a patient 18 and to provide thermal regulation to the patient, said mattress assembly comprising;
a mattress 26 containing a heat transfer medium,
a thermoelectric device 28 in heat transfer relationship with said mattress 26 and said heat transfer medium, and control means 30 activating said thermoelectric device 28 to cause said thermoelectric device to affect the temperature of said mattress 26.

2. A mattress assembly as claimed in Claim 1 wherein said thermoelectric device 28 provides cooling to said mattress 26.

3. A mattress assembly as claimed in Claim 1 wherein said thermoelectric device 28 provides both cooling and heating to said mattress 26.

4. A mattress assembly as claimed in Claims 1,2 or 3 wherein said heat transfer medium is a gel.

5. A mattress assembly as claimed in Claim 1 wherein said thermoelectric device 28 comprises a plurality of thermoelectric devices adapted to effect independently the temperature of said mattress 26 in separate segments 42.

6. A mattress assembly as claimed in Claim 6 wherein said thermoelectric device 28 simultaneously heats and cools one or more of said separate segments 42.

7. A method of heating and cooling an infant 18 comprising the steps of:
providing a mattress 26 underlying and supporting the infant 18 containing a heat conducting material;
providing a thermoelectric device 28 in heat transfer relationship with the mattress 26 and the heat conducting material;
providing an electrical circuit for causing the thermoelectric device 28 to provide temperature control to the mattress 26; and
controlling the electric circuit to cause the thermoelectric device 28 to cause the mattress 26 to reach a desired temperature.

8. A method of heating and cooling an infant as claimed in Claim 7 wherein the step of controlling the electric circuit causes the thermoelectric device 28 to either cool or heat the mattress 26.

9. A method of heating and cooling an infant as claimed in Claim 7 or 8 wherein the step of controlling the electric circuit causes the thermoelectric device 28 to simultaneously provide compartmentalized heating and cooling of the mattress 26.

10. A method of heating and cooling an infant as claimed in Claim 7 further including the steps of:
detecting the temperature of the mattress 26 and providing a electrical signal representative of the sensed temperature,
inputting a desired temperature of the mattress by a user input 40, and
controlling the electric circuit to establish the detected temperature of the mattress to be the temperature inputted by the user.
